# EUROPÄISCHE PATENTANMELDUNG

(11) **EP 1 214 934 A2**
(43) Veröffentlichungstag der Anmeldung: **19.06.2002**
(21) Anmeldenummer: 01127683.9
(22) Anmeldetag: 21.11.2001
(51) Int. Cl.: A61K 9/12, A23L 1/308

(54) **Oral verabreichbarer Stoff mit schwammartiger Struktur**

(30) Priorität: 15.12.2000 DE 10062624
(71) Anmelder: Ratjen, Werner, D-24220 Flintbek (DE); Willmen, Hans Rainer Prof. Dr. med., D-41515 Grevenbroich (DE)
(72) Erfinder: Ratjen, Werner, 24224 Flintbek (DE); Müller, Bernd W., Prof. Dr., 24220 Flintbek (DE)
(74) Vertreter: Vollmann, Heiko, Dipl.-Ing.

(57) **Zusammenfassung**

Ein oral verabreichbarer Stoff mit schwammartiger Struktur enthält folgende Hauptbestandteile:
A) ein oder mehrere anionische(s) Polymer(e)
B) ein oder mehrere kationische Polymer(e) und
C) pflanzliche Faserstoffe,
wobei Bestandteil (A) und Bestandteil (B) jeweils für sich quervernetzt sind, oder/und wobei die Bestandteile (A) und (B) miteinander quervernetzt sind.

## Beschreibung

Die Erfindung betrifft einen Stoff mit schwammartiger Struktur, der oral verabreicht werden kann und zur Gewichtsreduktion oder zur Behandlung der Fettsucht beim Menschen verwendet werden kann und der darüber hinaus zur Freisetzung von Wirkstoffen geeignet ist. Die Erfindung betrifft ferner ein Verfahren zur Herstellung derartiger Stoffe.

Die Behandlung des Übergewichts bzw. der Fettsucht beim Menschen kann zum einen durch chirurgische Eingriffe erfolgen, bei denen überschüssiges Körpergewebe entfernt wird. Der Abbau von überschüssigem Fettgewebe durch Steigerung des Energieverbrauchs, z. B. durch Sport, ist zwar grundsätzlich möglich, erfordert aber einen hohen Zeitaufwand, ist für bestimmte Patienten bzw. Patientinnen ungeeignet und führt nicht immer zum gewünschten Erfolg.

Zum anderen kann durch eine gezielte Verringerung der Aufnahme verwertbarer Nahrungsstoffe eine Gewichtsreduktion bewirkt werden, was aber eine gewisse Willensstärke bzw. ein gewisses Maß an Selbstbeherrschung bei den Patienten und Patientinnen voraussetzt.
Um die Patienten bzw. Patientinnen bei der Änderung ihres Nahrungsaufnahmeverhaltens zu unterstützen, können beispielsweise Appetitzügler eingesetzt werden, welche aufgrund ihrer pharmakologischen Wirkung eine Unterdrückung des Hungergefühls herbeiführen. Wegen ihrer Nebenwirkungen und der von ihnen ausgehenden Suchtgefahr ist vom Einsatz solcher Appetitzügler allerdings grundsätzlich abzuraten.

Andere Methoden zielen darauf ab, durch Einbringung voluminöser, quellfähiger, schwer oder nicht verdaulicher, brennwertarmer Stoffe in den Magen ein Völlegefühl zu erzeugen und auf diese Weise das Hungergefühl zu unterdrücken. Eine derartige Wirkung haben auch die sogenannten Ballaststoffe, wie z. B. pflanzliche Nahrungsfasern. Allerdings ist deren Verweilzeit im Magen relativ kurz, und sie fördern zudem die Darmperistaltik, so daß nur kurzzeitig eine Unterdrükkung des Hungergefühls bewirkt wird.

Aus US-A-4,401,682 und WO 90/01879 sind oral verabreichbare Mittel bekannt, welche fein vermahlene Cellulosefasern vermengt mit Gelatine enthalten. Im Magen bildet sich daraus durch Quellung eine gallertartige Masse mit undefinierter Raumstruktur, die relativ schnell abbaubar ist. Folglich muß ein solches Mittel in kurzen Zeitabständen eingenommen werden, um ein anhaltendes Sättigungsgefühl zu erzeugen. Aufgrund des damit verbundenen erhöhten Massendurchsatzes besteht die Gefahr des Auftretens von Nebenwirkungen. Zudem ist diese Methode aus Kostengründen und im Hinblick auf die Patienten-Compliance als nachteilig zu bewerten.

In EP 0 471 217 B1 werden oral verabreichbare Mittel zur Erzeugung eines Sättigungsgefühls beschrieben. Dabei handelt es sich um einen nach der Freisetzung im Magen volumenvergrößernden, nicht toxischen und brennstoffarmen Stoff, der sich in komprimiertem Zustand in einem Behältnis befindet, dessen Wandung im Magen auflösbar ist. Bevorzugt wird als volumenvergrößernder Stoff ein Celluloseschwamm verwendet; das Behältnis wird vorzugsweise durch eine Gelatinekapsel gebildet. Derartige Celluloseschwämme sind zwar kostengünstig herstellbar, haben jedoch den Nachteil, daß sie relativ hart sind und somit die Wandung der Verdauungsorgane mechanisch reizen können. Dies kann zumindest bei bestimmten Patienten zu Schmerzempfindungen bzw. zu Mißempfindungen führen. Zudem sind pathologische Veränderungen nach fortgesetzter Einnahme solcher Mittel nicht auszuschließen.

Aufgabe der vorliegenden Erfindung war es deshalb, einen Stoff bereitzustellen, der
- oral verabreichbar ist,
- als Mittel zur Gewichtsreduktion oder zur Freisetzung von Wirkstoffen im Magen geeignet ist,
- in wässrigen Medien, z. B. im Magensaft quellfähig bzw. zur Volumenvergrößerung befähigt ist,
- der keinen oder nur einen geringen Brennwert aufweist und der im Magen-Darm-Trakt nicht oder nur sehr langsam abgebaut wird,
- der nicht toxisch und weitgehend frei von Nebenwirkungen ist und das Verdauungssystem der Patienten nicht übermässig belastet,
- der die vorstehend genannten Nachteile vermeidet, insbesondere hinsichtlich der Kosten und Patienten-Compliance.

Des weiteren bestand die Aufgabe darin, ein Verfahren anzugeben, mit dessen Hilfe ein derartiger Stoff kostengünstig und auf einfache Weise produziert werden kann.

Überraschenderweise wurde gefunden, daß die gestellten Aufgaben durch einen Stoff mit schwammartiger Struktur gemäß Anspruch 1 und durch ein Verfahren gemäß Anspruch 22 gelöst werden, sowie durch die in den Unteransprüchen beschriebenen Ausführungsformen.

Erfindungsgemäß wird zur Lösung der Aufgabe ein Stoff mit schwammartiger Struktur bereitgestellt, der als Hauptbestandteile ein oder mehrere anionische(s) Polymer(e) (Komponente A), ein oder mehrere kationische(s) Polymer(e) (Komponente B) und pflanzliche Faserstoffe (Komponente C) enthält, wobei Bestandteil (A) und Bestandteil (B) jeweils für sich quervernetzt sind, oder/und wobei die Bestandteile (A) und (B) miteinander quervernetzt sind.

Diese erfindungsgemäßen schwammartigen Strukturen sind elastisch und quellbar bzw. zur Volumenvergrößerung befähigt. Aufgrund der Quervernetzung der beteiligten Polymere ist deren Löslichkeit herabgesetzt und ein enzymatischer Abbau im Verdauungstrakt erschwert. Die Quellbarkeit bzw. Wasseraufnahmefähigkeit wird durch die Anwesenheit von Pflanzenfasern zusätzlich gesteigert.

Dank der elastischen Eigenschaften lassen sich die schwammartigen Strukturen gut durch mechanischen Druck komprimieren, so daß sie in komprimierter Form oral verabreicht werden können. Durch die schwammartige, elastische Struktur wird auch eine mechanische Reizung der Verdauungsorgane weitgehend ausgeschlossen.
Da bei der Herstellung, wie nachfolgend beschrieben, fast ausschließlich auf nichttoxische bzw. lebensmittelgeeignete Ausgangsstoffe zurückgegriffen werden kann, sind die erhaltenen schwammartigen Produkte gesundheitlich unbedenklich und Nebenwirkungen grundsätzlich nicht zu erwarten.

Aufgrund der schwammartigen, quellfähigen Struktur und der verzögerten Abbaubarkeit wird eine verlängerte Verweildauer im Magen bewirkt, so daß eine anhaltende Unterdrückung des Hungergefühls hervorgerufen wird. Aufgrund der verlängerten Verweilzeit im Magen können die erfindungsgemäßen schwammartigen Strukturen auch vorteilhaft zur Verabreichung von pharmazeutischen Wirkstoffen, Vitaminen, Mineralstoffen und anderen Stoffen, die überwiegend durch die Magenschleimhaut aufgenommen werden, verwendet werden. Hierbei ist es zusätzlich von Vorteil, daß die in das Schwammgerüst eingelagerten Wirkstoffe nach erfolgter Quellung verzögert oder in kontrollierter Weise nach außen abgegeben werden.

Als anionische Polymere (Komponente A) eignen sich vor allem solche aus der Gruppe der wasserlöslichen Polypeptide oder/und aus der Gruppe der wasserlöslichen anionischen Polysaccharide. Aber auch andere wasserlösliche, quervernetzbare anionische Polymere können in Betracht kommen.
Als wasserlösliche Proteine für die Komponente (A) kommen grundsätzlich alle lebensmittelgeeigneten Proteine oder Proteingemische in Frage. Die Polypeptide liegen in den erfindungsgemäßen schwammartigen Strukturen in chemisch quervernetzter Form vor, oder zumindest in teilweise vernetzter Form.
Besonders bevorzugt wird hierfür Gelatine verwendet, insbesondere Typ-B-Gelatine. Unabhängig davon, welches Polypeptid oder Polypeptidgemisch verwendet wird, sollte dessen isoelektrischer Punkt niedriger als 6,5 sein, vorzugsweise niedriger als 5,5, um eine Komplexbildung mit der Komponente (B) zu begünstigen. Als weiteres Beispiel für ein geeignetes Polypeptid ist Kollagen zu nennen, das allerdings relativ teuer in der Herstellung ist.
Aus der Gruppe der wasserlöslichen anionischen Polysaccharide werden beispielsweise Alginsäure und Pektin genannt.

Als kationische Polymere für die Komponente (B) eignen sich grundsätzlich solche, die chemisch quervernetzbar sind, und die in der Lage sind, zusammen mit der Komponente (A) Schäume zu bilden. Vorzugsweise werden sie aus der Gruppe der quervernetzbaren kationischen wasserlöslichen Polymere, insbesondere aus der Gruppe der aminogruppenhaltigen Polysaccharide, ausgewählt, bei welchen z. B. durch bifunktionelle Aldehyde eine Quervernetzung erzeugt werden kann. Bei den genannten Polymeren kann es sich um natürliche oder partialsynthetische Polymere handeln (dies gilt entsprechend auch für die Polymere der Komponente A). Auch Proteine, welche die genannten Eigenschaften aufweisen, sind grundsätzlich als Komponente (B) geeignet, z. B. Typ-A-Gelatine.
Als "quervernetzbar" werden vorzugsweise solche Polymere verstanden, die funktionelle Gruppen (z. B. Amino-, Carboxyl- oder Hydroxylgruppen) aufweisen, welche kovalente Bindungen mit bifunktionellen oder multifunktionellen Quervernetzungs-Agenzien eingehen können, wobei es zur Bildung von intermolekularen Brücken kommt.

Besonders geeignet als Bestandteil (B) ist Chitosan, ein lineares Polysaccharid, welches durch chemische Deacetylierung von Chitin erhalten wird. Chitosan wird in der Regel aus Schalen von Krebsen oder Krabben gewonnen und ist ein kommerziell erhältliches Produkt, z. B. in Pulver- oder Flockenform. Chitosan ist in unterschiedlichen Deacetylierungsgraden erhältlich; vorzugsweise wird Chitosan mit einem Deacetylierungsgrad von mindestens 70 % verwendet; Chitosan mit einem Deacetylierungsgrad von mindestens 90 % ist besonders bevorzugt. Der Deacetylierungsgrad des Chitosans beeinflußt die Härte bzw. Elastizität der erfindungsgemäßen Schwämme. Mit hochdeacetyliertem Chitosan werden weichere und elastischere schwammartige Strukturen erhalten als mit weniger stark deacetyliertem Chitosan. Falls erforderlich, können auch Mischungen von Chitosanzubereitungen mit unterschiedlichen Deacetylierungsgraden eingesetzt werden.

Der mengenmäßige Anteil des vernetzten Polysaccharids bzw. der Komponente (B) ist vorzugsweise niedriger als der Anteil des vernetzten Proteins bzw. der Komponente (A). Der letztgenannte Anteil entspricht vorzugsweise dem 5- bis 15-fachen, besonders bevorzugt dem 7- bis 10-fachen des Polysaccharid-Anteils bzw. des Anteils des Bestandteils (B).

Allgemein richtet sich der mengenmäßige Anteil der Komponente (A) bzw. (B) nach der Anzahl der funktionellen Gruppen, die bis zum Ladungsausgleich miteinander reagieren (unter Bildung eines Komplexes).

Die erfindungsgemäßen schwammartigen Stoffe enthalten als dritte Hauptkomponente (Bestandteil C) pflanzliche Faserstoffe, deren Anwesenheit in Kombination mit den Komponenten (A) und (B) von wesentlicher Bedeutung für die Vorteile und Eigenschaften der schwammartigen Strukturen hinsichtlich ihrer Verwendung als Mittel zur Gewichtsreduktion ist. Grundsätzlich können alle pflanzlichen Faserstoffe verwendet werden, die für die Verwendung in Lebensmitteln geeignet sind, beispielsweise sogenannte Ballaststoffe. Bevorzugt werden dabei aus Getreidearten gewonnene Faserstoffe, z. B. Weizenpflanzenfasern eingesetzt. Diese sollten möglichst feinfaserig sein, beispielsweise mit einer durchschnittlichen Faserlänge von weniger als 150 µm und einer durchschnittlichen Faserdicke von weniger als 50 µm.

Der mengenmäßige Anteil des Bestandteils (C) (pflanzliche Faserstoffe) ist vorzugsweise so bemessen, daß er dem 0,1-bis 5-fachen, besonders bevorzugt dem 0,5- bis 1,5-fachen des Anteils der Komponente (A) bzw. des Polypeptid-Anteils entspricht, bezogen auf die Masse in g.

Um die mechanischen Eigenschaften, wie Härte und Elastizität, der erfindungsgemäßen Stoffe mit schwammartiger Struktur zu beeinflussen, bzw. um während der Herstellung die Aufschäumbarkeit der Polymerlösungen, insbesondere der Polypeptidlösung, zu verbessern, können Weichmacher zugesetzt werden. Dabei eignen sich vorzugsweise Stoffe aus der Gruppe der Polyole (z. B. Glycerol, Propylenglykol, Polyethylenglykol), Zuckeralkohole, Monosaccharide, Fettsäuren, Fettsäureester, Hydroxycarbonsäureester (z. B. Citronensäure-, Milchsäure-, Weinsäureester) und Salze von Hydroxycarbonsäureestern (z. B. Citrate, Lactate, Tartrate). Auch mit anorganischen Salzen kann eine weichmachende Wirkung erzielt werden. Grundsätzlich kommen aus den vorgenannten Stoffen bzw. Stoffgruppen solche in Betracht, die nichttoxisch bzw. für die Verwendung in Lebensmitteln geeignet sind. Besonders bevorzugt wird Sorbitol verwendet, sowie Mannitol oder NaCl. Selbstverständlich können auch Kombinationen der genannten Weichmacher eingesetzt werden.
Anstelle von Sorbitol können auch solche Polyole verwendet werden, die beispielsweise durch Hydrierung eines Stärkehydrolysats erhalten wurden.
Der Weichmachergehalt im schwammartigen Stoff beträgt vorzugsweise 0,5-25 Gew.-%, vorzugsweise 1-10 Gew.-%.

Bei der Herstellung der erfindungsgemäßen Schwämme werden die Polymere, gegebenenfalls mit weiteren Zusatzstoffen, zunächst gelöst, vorzugsweise in einer verdünnten Säure. Als Säuren kommen vorzugsweise Monocarbonsäuren, wie z. B. Ameisensäure, Essigsäure, Propionsäure, oder Hydroxycarbonsäuren, wie z. B. Milchsäure, Äpfelsäure, Citronensäure und Weinsäure, in Betracht, aber auch mineralische Säuren, wie z. B. HCl. Der Restsäuregehalt im getrockneten Schwamm ist abhängig vom Säuregehalt der Lösungen und von der Flüchtigkeit der eingesetzten Säuren; er liegt typischerweise im Bereich von 0,1 bis 20 Gew.-%.

Zusätzlich zu den oben genannten Inhaltsstoffen können die erfindungsgemäßen schwammartigen Stoffe noch weitere Inhaltsstoffe enthalten. So kann es z. B. vorteilhaft sein, wenn diese Schwammstrukturen z. B. Arzneimittelwirkstoffe, Vitamine oder Mineralstoffe enthalten, die während der Verweilzeit, nach erfolgter Quellung, im Magen langsam bzw. verzögert freigesetzt werden, und die überwiegend über die Magenschleimhaut aufgenommen werden können.

Als Arzneimittelwirkstoffe kommen hierfür insbesondere solche in Frage, welche die beabsichtigte Gewichtsreduktion unterstützen oder eine Unterdrückung des Hungergefühls bewirken, falls dies im Einzelfall für vorteilhaft oder erforderlich gehalten wird, beispielsweise Appetitzügler wie Pseudoephedrin, Amfepramon, Mefenorex, Fenproporex. Aber auch andere Arzneistoffe, die vornehmlich über die Magenschleimhaut resorbiert werden, lassen sich vorteilhaft mit den erfindungsgemäßen schwammartigen Stoffen verabreichen. Dabei kann es sich sowohl um Magen- oder Magen-Darm-Mittel handeln, als auch um systemisch wirkende Stoffe.

Gemäß Anspruch 1 enthalten die erfindungsgemäßen schwammartigen Stoffe quervernetzte anionische bzw. kationische Polymere. Die Vernetzung wird bevorzugt auf chemischem Wege mit Hilfe von bifunktionellen Aldehyden als Vernetzungsmittel erzeugt, wie z. B. Glutaraldehyd oder Diisocyanaten. Im Endprodukt ist freies Vernetzungsmittel, das möglicherweise gesundheitsgefährdend sein könnte, nicht mehr oder höchstens in geringsten Spuren vorhanden.

Die erfindungsgemäßen schwammartigen Stoffe weisen im Endzustand nach Trocknung bzw. nach Trocknung und Pressung einen Wassergehalt von weniger als 15 Gew.-%, vorzugsweise weniger als 12, besonders bevorzugt von weniger als 5 Gew.- % auf.
Sie sind in wässrigen Medien quellfähig, wobei sie vorzugsweise mindestens eine ihrem eigenen Gewicht entsprechende Wassermenge aufnehmen können. Im menschlichen Verdauungstrakt werden sie nur sehr langsam degradiert oder unverdaut ausgeschieden.

Die Erfindung schließt ferner auch solche Ausführungsformen mit ein, bei welchen die erfindungsgemäßen Stoffe mit schwammartiger Struktur in komprimiertem Zustand vorliegen, oder in komprimiertem Zustand in ein Behältnis eingebracht sind, welches aufgrund seiner Form und Zusammensetzung zur oralen Verabreichung geeignet ist, und dessen Wandung im Magen aufgelöst wird, wodurch der schwammartige Stoff freigesetzt wird und aufquillt.
Als Behältnis werden vorzugsweise Hartgelatinekapseln, besonders bevorzugt zweiteilige Hartgelatinekapseln (Gelatine-Steckkapseln) verwendet, wie sie auch als Einhüllung für Medikamente verwendet werden. Besonders bevorzugt werden Gelatine-Steckkapseln der Größe 0, 00 oder 000 mit einem Fassungsvermögen von 0,4 bis 1,5 cm³ verwendet.

Die Erfindung betrifft auch ein Verfahren zu Herstellung der vorstehend beschriebenen Stoffe mit schwammartiger Struktur. Das Verfahren beruht im wesentlichen darauf, daß zunächst in einem Schritt (a) eine wässrige Lösung eines oder mehrerer vernetzbarer Polymere der Komponente (A) (z. B. Polypeptid oder Polypeptidgemisch) hergestellt und in dieser Lösung eine chemische Vernetzungsreaktion durchgeführt wird ("Mischung 1"). Diese Lösung enthält außerdem pflanzliche Faserstoffe (Komponente (C))
Auf entsprechende Weise wird in einem Schritt (b) eine wässrige Lösung eines oder mehrerer kationischer Polymere (Komponente B; z. B. vernetzbares Polysaccharid oder Polysaccharid-Gemisch) hergestellt und dieses ebenfalls einer Vernetzungsreaktion unterzogen ("Mischung 2").

Anschließend werden beide Lösungen bzw. Ansätze (Mischung 1, Mischung 2) vereinigt (Schritt (c)) (Schritt (d)) und aufgeschäumt, was beispielsweise durch heftiges mechanisches Rühren oder auf andere, dem Fachmann bekannte Weise erfolgen kann, z. B. durch Einrühren (Homogenisieren) von Luft oder Gasen (z. B. Kohlendioxid).

Schließlich wird nach Abfüllen des Schaums in geeignete Behälter oder Formen durch Trocknung zur Entfernung des Wasseranteils und der sonstigen flüchtigen Anteile ein fester, elastischer Stoff mit schwammartiger Struktur erhalten. Die Trocknung erfolgt vorzugsweise bei 25-80 °C, besonders bevorzugt bei 40-60 °C. Sie kann mit Hilfe einer Wärmekammer oder eines Trockenofens, oder durch vakuumtrocknung bewirkt werden; auch Gefriertrocknungsverfahren können zum Einsatz kommen.

Der Polypeptidanteil bzw. Anteil der Komponente (A) in der wässrigen Mischung 1 beträgt vorzugsweise 0,1 bis 10 Gew.- %, besonders bevorzugt 2,0 bis 5,0 Gew.-%. Für den Anteil der pflanzlichen Faserstoffe gelten dieselben genannten Bereichsangaben. Als Polypeptidgemisch wird, wie vorstehend beschrieben, Gelatine-Typ-B besonders bevorzugt.
Die Herstellung der Mischung 1 erfolgt unter Erwärmung auf 40-70 °C, vorzugsweise auf 50-65 °, wobei der Auflösungsvorgang durch Rühren beschleunigt wird. Nach vollständiger Auflösung wird die Lösung auf Raumtemperatur abkühlen lassen (ca. 20-25 °C).

Der Anteil der Komponente (B) in der wässrigen Mischung 2 beträgt vorzugsweise 0,1 bis 10 Gew.-%, besonders bevorzugt 0,5 bis 5,0 Gew.-%. Die Herstellung der Mischung 2 erfolgt wie für Mischung 1 beschrieben, jedoch ohne Erwärmung der Mischung.
Ein bevorzugtes kationisches Polysaccharid ist, wie vorstehend beschrieben, Chitosan.

Vorzugsweise werden zur Herstellung der wässrigen Lösungen 1 und 2 verdünnte Säuren verwendet; hierfür geeignete Stoffe wurden bereits weiter oben genannt. Die Konzentration der Säure(n) beträgt jeweils ca. 0,1 bis 5,0 Gew.-%.

Im Falle der Mischung 1 wird besonders bevorzugt Milchsäure, im Falle der Mischung 2 besonders bevorzugt Essigsäure verwendet.

Sowohl Mischung 1 als auch Mischung 2 enthalten jeweils ein Vernetzungsreagenz, so daß während der Schritte (a) bzw. (b) eine Vernetzung der Polymermoleküle stattfindet. Diese Vernetzungsreaktion kann auch noch nach erfolgter Schaumbildung bzw. während des Trocknungsvorganges andauern.

Nach der oben beschriebenen bevorzugten Ausführungsform werden die Komponenten (A) und (B) in getrennten Reaktionsansätzen vernetzt, um zu vermeiden, daß frühzeitige, nicht erwünschte Vernetzungen entstehen. Dennoch kann es in bestimmten Fällen auch vorteilhaft sein, wenn die Vernetzung der beiden Komponenten in einem gemeinsamen Reaktionsansatz durchgeführt wird, so daß die Polymerketten der Komponenten (A) und (B) auch miteinander vernetzt werden.

Als Vernetzungsmittel werden bevorzugt bifunktionelle Aldehyde verwendet, besonders bevorzugt Glutardialdehyd. Dieser wird üblicherweise als 5 %ige wässrige Lösung eingesetzt. Die Konzentration des Glutardialdehyds beträgt in Schritt a) 0,05 bis 5,0 Gew.-%, bevorzugt 0,1 bis 2,0 Gew.-%, bezogen auf die Menge der eingesetzten Komponente (A), und in Schritt b) 0,1 bis 10,0 Gew.-%, bevorzugt 1,0 bis 5,0 Gew.- %, bezogen auf die Menge der eingesetzten Komponente (B). Eine Erhöhung der Vernetzer-Konzentration bewirkt, daß die Härte der so erzeugten Schwämme zunimmt.
Als weitere Vernetzungsmittel komnen andere Dialdehyde (z. B: Succindialdehyd), oder Diisocyanate (z. B. Hexamethylendiisocyanat) oder Carbodiimide in Frage.
Die Vernetzungsreaktion wird bevorzugt bei Raumtemperatur (ca. 18-25 °C) oder leicht erhöhten Temperaturen (ca. 25-45 °C, bevorzugt 30-40 °C) und unter ständigem Durchmischen des Reaktionsansatzes durchgeführt, über einen Zeitraum von mindestens einer halben Stunde, vorzugsweise als Über-Nacht-Reaktion (ca. 8-16 h).

Wegen der möglichen Gesundheitsgefährdung, die von chemischen Vernetzungsmitteln ausgeht, muß gewährleistet sein, daß das Endprodukt (getrockneter Schwamm) weitestgehend frei von Rest-Anteilen solcher Stoffe ist. Dies kann z. B. im Falle des Glutardialdehyds durch geeignete, dem Fachmann bekannte Nachweismethoden überprüft werden.

Der Zusatz von Arzneimittelwirkstoffen, Vitaminen, Mineralstoffen etc. erfolgt vorzugsweise nach der Quervernetzungsreaktion, aber vor dem Aufschäumen. Falls eine Wechselwirkung dieser Stoffe mit dem Vernetzungs-Agens nicht zu erwarten ist, können diese auch schon während der Herstellung der Polymerlösungen zugesetzt werden.

Durch Vereinigung der Lösungen 1 und 2 (Schritt c) wird ein Komplex aus vernetzten Proteinmolekülen (Komponente A) und Polysaccharidmolekülen (Komponente B) gebildet. Diese Komplexbildung wird durch einen sauren pH-Bereich begünstigt (vorzugsweise pH 4 bis pH 6,5).

Wie bereits beschrieben, weisen die erfindungsgemäßen schwammartigen Stoffe vorzugsweise einen Gehalt an Weichmacher(n) auf. Diese(r) Weichmacher werden ebenfalls den Lösungen 1 und/oder 2 zugesetzt.

Grundsätzlich ist bei der Auswahl aller Inhaltsstoffe zu beachten, daß diese für Lebensmittelzwecke geeignet sind, insbesondere nicht toxisch sind und nicht mikrobiologisch kontaminiert sind. Auch müssen die Inhaltsstoffe den für Lebensmittel bzw. Arzneimittel erforderlichen Reinheitsgrad aufweisen.

Die Herstellung der erfindungsgemäßen schwammartigen Strukturen wird an Hand der nachfolgenden Beispiele näher erläutert:

### Beispiel 1

### Schritt a):

### Herstellung von "Mischung 1" (berechnet auf 1000 g Masse)

| | |
|---|---|
| 24,94 g | Gelatine |
| 548,62 g | Wasser |
| 0,83 g | Milchsäure |
| 3,33 g | Glutardialdehyd-Lösung (5%ig) |
| 28,26 g | Weizenfaserstoff (durchschnittl. Faserlänge: 80 µm; Faserdicke: 20 mm) |
| 14,96 g | Sorbitol und |
| 6,65 g | Gluten |

werden unter ständigem Rühren auf 50-65 °C erwärmt (Dauer: ca. 2 h); anschließend wird der Ansatz wieder auf ca. 25 °C abgekühlt (Mischung 1 insgesamt: 627,59 g).

### Schritt b): Herstellung von "Mischung 2"

| | |
|---|---|
| 3,33 g | Chitosan |
| 365,75 g | Wasser |
| 1,66 g | Essigsäure |
| 1,66 g | Glutardialdehyd-Lösung (5%ig) |

werden ohne Erwärmung (Raumtemperatur) gerührt (ca. 2 h). (Mischung 2 insgesamt: 372,40 g).

### Schaumherstellung (Schritte c) und d)):

Beide "Lösungen" werden vereinigt (Gesamtmenge: 1000 g) und in einer Aufschlagmaschine mit ca. 10.000 Umdrehungen/min aufgeschlagen, bis eine schaumähnliche Masse mit einem guten Volumen entstanden ist (Dauer: ca. 20-30 min).

### Trocknung:

Die Schaummasse wird in einen Behälter (z. B. flache Form) gefüllt, wobei die Füllhöhe bzw. die Schichtdicke nach Bedarf festgelegt wird. Anschließend wird das im Behälter befindliche Produkt in einer Wärmekammer oder in einem Trokkenofen bei 40-60 °C getrocknet. In Abhängigkeit von der Stärke und Höhe bzw. Schichtdicke der Schaummasse dauert die Trocknung ca. 3-5 Tage.

### Weiterverarbeitung des getrockneten Schaums (Schwamm):

Nach der Trocknung werden die schwammartigen Tafeln oder Platten, die einen Feuchtigkeitsgehalt von ca. 8 % aufweisen, in Streifen mit einer Breite von 3,5 cm geschnitten. Die Länge der Streifen ist durch das Behältermaß vorgegeben.
Die so erzeugten Streifen werden unter sehr hohem Druck in einer Spezialmaschine so stark gepreßt, daß im Querschnitt viereckige Streifen (ca. 0,5 x 0,5 cm) entstehen. Diese komprimierten Streifen werden danach auf eine Länge von 1,5 -2,0 cm zugeschnitten und in eine Gelatinekapsel (Größe 00) eingebracht.

Die Dichte eines gepreßten Stückes beträgt beispielsweise 0,48 g/cm³.
Der Wassergehalt für ein getrocknetes, gepreßtes Stück beträgt beispielsweise 10,50 %. Die Wasseraufnahme beträgt 1,120 %.
Der Brennwert des getrockneten, fertigen Produkts liegt bei 218 kcal/100 g.

### Beispiel 2:

### Schritt a): Herstellung von "Lösung 1":

| | |
|---|---|
| 300 g | Gelatine (Typ B; Bloom-Wert: 180). |
| 6000 g | Wasser |
| 10 g | Milchsäure |
| 35 g | 5%ige Glutardialdehyd-Lösung |
| 35 g | Sorbitol und |
| 250 g | Weizenfaserstoff (durchschnittl. Faserlänge: 80 µm; Faserdicke: 20 mm) |

werden unter ständigem Rühren auf 50-65 °C erwärmt (Dauer: ca. 2 h); anschließend wird der Ansatz wieder auf ca. 25 °C abgekühlt.

### Schritt b): Herstellung von "Lösung 2"

| | |
|---|---|
| 40 g | Chitosan (Deacetylierungsgrad: 93,0 %) |
| 20 g | Essigsäure |
| 4000 g | Wasser |
| 20 g | 5%ige Glutardialdehyd-Lösung |

werden ohne Erwärmung (Raumtemperatur) gerührt (ca. 2 h). Die weitere Verarbeitung erfolgte wie oben für Beispiel 1 angegeben.

Die erfindungsgemäßen Stoffe mit schwammartiger Struktur sowie oral verabreichbare Kapseln, die diese Stoffe enthalten, eignen sich aufgrund der vorstehend beschriebenen Eigenschaften in vorteilhafter Weise als Mittel zur Unterdrückung des Hungergefühls, zur Gewichtsreduktion oder zur Behandlung der Fettsucht beim Menschen.

Sie können ferner als orale Darreichungsformen zur Verabreichung von Vitaminen oder Mineralstoffen oder von Arzneimittelwirkstoffen, welche überwiegend über die Magenschleimhaut resorbiert werden, verwendet werden. Dabei besteht der Vorteil der erfindungsgemäßen schwammartigen Stoffe darin, daß sie einerseits eine verlängerte Verweilzeit im Magen aufweisen, andererseits zur verzögerten oder kontrollierten Wirkstoffabgabe befähigt sind. Sie eignen sich deshalb insbesondere dann, wenn pharmazeutische Wirkstoffe, Vitamine, Mineralstoffe oder andere resorbierbare oder physiologisch wirksame Stoffe im Magen über einen längeren Zeitraum freigesetzt werden sollen ("stomach targeting of drugs").

## Patentansprüche

1. Oral verabreichbarer Stoff mit schwammartiger Struktur, welcher folgende Hauptbestandteile enthält:
A) ein oder mehrere anionische(s) Polymer(e)
B) ein oder mehrere kationische Polymer(e) und
C) pflanzliche Faserstoffe,
wobei Bestandteil (A) und Bestandteil (B) jeweils für sich quervernetzt sind, oder/und wobei die Bestandteile (A) und (B) miteinander quervernetzt sind.

2. Stoff nach Anspruch 1, **dadurch gekennzeichnet, daß** Bestandteil (A) aus der Gruppe der wasserlöslichen Polypeptide oder/und aus der Gruppe der wasserlöslichen anionischen Polysaccharide ausgewählt ist.

3. Stoff nach Anspruch 2, **dadurch gekennzeichnet, daß** er als Bestandteil (A) chemisch quervernetzte Gelatine, bevorzugt Typ-B-Gelatine enthält.

4. Stoff nach Anspruch 2 oder 3, **dadurch gekennzeichnet, daß** der isoelektrische Punkt der Polypeptide kleiner als 6,5, vorzugsweise kleiner als 5,5 ist.

5. Stoff nach einem der Ansprüche 1 bis 4, **dadurch gekennzeichnet, daß** Bestandteil (B) aus der Gruppe der guervernetzbaren kationischen wasserlöslichen Polymere ausgewählt ist und ein natürliches oder partialsynthetisches Polymer ist.

6. Stoff nach Anspruch 5, **dadurch gekennzeichnet, daß** Bestandteil (B) aus der Gruppe der aminogruppenhaltigen Polysaccharide ausgewählt ist, wobei Chitosan besonders bevorzugt ist.

7. Stoff nach einem oder mehreren der vorangehenden Ansprüche, **dadurch gekennzeichnet, daß** er als Bestandteil (C) lebensmittelgeeignete Faserstoffe enthält, vorzugsweise aus Getreidearten gewonnene Faserstoffe, besonders bevorzugt Weizenfasern.

8. Stoff nach einem oder mehreren der vorangehenden Ansprüche, **dadurch gekennzeichnet, daß** er zusätzlich einen oder mehrere Mineralstoffe enthält.

9. Stoff nach einem oder mehreren der vorangehenden Ansprüche, **dadurch gekennzeichnet, daß** er zusätzlich ein oder mehrere Vitamine enthält.

10. Stoff nach einem oder mehreren der vorangehenden Ansprüche, **dadurch gekennzeichnet, daß** er zusätzlich mindestens einen Arzneimittel-Wirkstoff, enthält, vorzugsweise aus der Gruppe der magenresorbierbaren Wirkstoffe oder aus der Gruppe der Appetitzügler.

11. Stoff nach einem oder mehreren der vorangehenden Ansprüche, **dadurch gekennzeichnet, daß** er zusätzlich einen Gehalt an einem oder mehreren Weichmachern aufweist, wobei der/die Weichmacher vorzugsweise aus der anorganische Salze, Monosaccharide, Polyole, Zuckeralkohole, Fettsäuren und Fettsäureester, Hydroxycarbonsäureester und Salze von Hydroxycarbonsäuren umfassenden Gruppe ausgewählt ist/sind, besonders bevorzugt aus der Sorbitol, Mannitol, Glycerol, Propylenglykol, Polyethylenglykol und NaCl umfassenden Gruppe.

12. Stoff nach Anspruch 11, **dadurch gekennzeichnet, daß** der Weichmachergehalt 0,5-25 Gew.-%, vorzugsweise 1-10 Gew.-% beträgt.

13. Stoff nach einem oder mehreren der vorangehenden Ansprüche, **dadurch gekennzeichnet, daß** er zusätzlich einen Gehalt an mindestens einer mineralischen oder organischen Säure aufweist, wobei die Säure(n) vorzugsweise aus der HCl, Ameisensäure, Essigsäure, Propionsäure, Milchsäure, Äpfelsäure, Citronensäure und Weinsäure umfassenden Gruppe ausgewählt ist/sind, und wobei Milchsäure und Essigsäure besonders bevorzugt sind.

14. Stoff nach einem oder mehreren der vorangehenden Ansprüche, **dadurch gekennzeichnet, daß** die chemische Quervernetzung mittels eines bifunktionellen Aldehyds erzeugt ist, besonders bevorzugt mittels Glutardialdehyd, oder mittels eines Diisocyanats.

15. Stoff nach einem oder mehreren der vorangehenden Ansprüche, **dadurch gekennzeichnet, daß** sein Wassergehalt weniger als 15 Gew.-%, vorzugsweise weniger als 12 Gew.-%, besonders bevorzugt weniger als 5 Gew.-% beträgt.

16. Stoff nach einem oder mehreren der vorangehenden Ansprüche, **dadurch gekennzeichnet, daß** der mengenmäßige Anteil des Bestandteils A) (Polypeptide) dem 5- bis 15-fachen, vorzugsweise dem 7- bis 10-fachen des Polysaccharid-Anteils (Bestandteil B)) entspricht, jeweils bezogen auf die Masse in g.

17. Stoff nach einem oder mehreren der vorangehenden Ansprüche, **dadurch gekennzeichnet, daß** der mengenmäßige Anteil des Bestandteils C) (pflanzliche Faserstoffe) dem 0,1-bis 5-fachen, besonders bevorzugt dem 0,5- bis 1,5-fachen des Polypeptid-Anteils entspricht, bezogen auf die Masse in g.

18. Stoff nach einem oder mehreren der vorangehenden Ansprüche, **dadurch gekennzeichnet, daß** bei seiner Herstellung der Bestandteil (A) zusammen mit pflanzlichen Fasern (Bestandteil C), und dem Bestandteil (B), welche jeweils in einer wässrigen Lösung oder Suspension vorliegen, in getrennten Reaktionen chemisch vernetzt werden, und daß anschließend die beiden Ansätze vereinigt werden, aufgeschäumt und nachfolgend getrocknet werden.

19. Stoff nach einem oder mehreren der vorangehenden Ansprüche, **dadurch gekennzeichnet, daß** er in wässrigen Medien quellfähig ist und vorzugsweise eine erhöhte Verweilzeit im Magen aufweist, daß er im menschlichen Verdauungstrakt nur langsam degradiert wird, und daß er einen niedrigen physiologischen Brennwert aufweist.

20. Stoff nach einem oder mehreren der vorangehenden Ansprüche, **dadurch gekennzeichnet, daß** er in komprimierter Form vorliegt.

21. Stoff nach Anspruch 20, **dadurch gekennzeichnet, daß** von einer für die orale Verabreichung geeigneten Hartgelatine-Kaspel umschlossen ist.

22. Verfahren zur Herstellung eines Stoffes mit schwammartiger Struktur nach einem oder mehreren der Ansprüche 1 bis 19, welcher als Hauptbestandteile
A) ein oder mehrere quervernetzte anionische Polymere
B) ein oder mehrere quervernetzte kationische Polymere und
C) pflanzliche Faserstoffe enthält,
wobei das Verfahren folgende Schritte aufweist:
a) Herstellung einer den Bestandteil A, chemisches Vernetzungsmittel und pflanzlichen Faserstoff enthaltenden wässrigen Mischung 1 unter Erwärmung auf 40-70 °C und ständigem Rühren;
b) Herstellung einer den Bestandteil (B) und Vernetzungsmittel enthaltenden wässrigen Mischung 2 unter ständigem Rühren;
c) Vereinigung der Mischungen 1 und 2;
d) Erzeugung eines Schaums;
e) Abfüllen des Schaums in Behälter und nachfolgende Trocknung bei 25-80 °C, vorzugsweise bei 40-60 °C.

23. Verfahren nach Anspruch 22, **dadurch gekennzeichnet, daß** in Schritt a) Gelatine als Bestandteil (A) und in Schritt b) Chitosan als Bestandteil (B) eingesetzt wird.

24. Verfahren nach Anspruch 22 oder 23, **dadurch gekennzeichnet, daß** in Schritt a) mindestens eine Säure zugesetzt wird, welche vorzugsweise aus den in Anspruch 13 genannten Säuren ausgewählt ist, besonders bevorzugt Milchsäure.

25. Verfahren nach einem der Ansprüche 22-24, **dadurch gekennzeichnet, daß** in Schritt b) mindestens eine Säure zugesetzt wird, welche vorzugsweise aus den in Anspruch 13 genannten Säuren ausgewählt ist, besonders bevorzugt Essigsäure.

26. Verfahren nach einem der Ansprüche 22-25, **dadurch gekennzeichnet, daß** in Schritt a) ein Weichmacher zugesetzt wird, vorzugsweise aus der in Anspruch 11 genannten Gruppe.

27. Verfahren nach einem oder mehreren der Ansprüche 22-26, **dadurch gekennzeichnet, daß** als chemisches Vernetzungsmittel ein bifunktioneller Aldehyd verwendet wird, vorzugsweise Glutardialdehyd, besonders bevorzugt in Form einer 5%igen Lösung (Gew.-%), oder ein Diisocyanat.

28. Verfahren nach Anspruch 27, **dadurch gekennzeichnet, daß** in Schritt a) 0,05 bis 5,0 Gew.-%, bevorzugt 0,1 bis 2,0 Gew.-% Glutardialdehyd, bezogen auf die Menge des anionischen Polymers, und in Schritt b) 0,1 bis 10,0 Gew.-%, bevorzugt 1,0 bis 5,0 Gew.-%, bezogen auf die Menge des kationische Polymers, zugesetzt werden.

29. Verfahren nach einem oder mehreren der Ansprüche 22-28, **dadurch gekennzeichnet, daß** der Schaum in Schritt e) in Form schichtförmiger Platten erzeugt wird, die in Streifen geschnitten werden, welche sodann mittels mechanischem Druck komprimiert und wahlweise schließlich in komprimiertem Zustand einzeln in Hartgelatinekapseln eingebracht werden.

30. Verwendung eines Stoffes nach einem oder mehreren der Ansprüche 1-21 oder eines Verfahrensproduktes nach einem oder mehreren der Ansprüche 22-29 als oral zu verabreichendes Mittel zur Unterdrückung des Hungergefühls, zur Gewichtsreduktion oder zur Behandlung der Fettsucht beim Menschen.

31. Verwendung eines Stoffes nach einem oder mehreren der Ansprüche 1-21 oder eines Verfahrensproduktes nach einem oder mehreren der Ansprüche 22-29 als orale Darreichungsform zur Freisetzung von Arzneimittelwirkstoffen, Vitaminen oder Mineralstoffen im Magen, vorzugsweise zur länger anhaltenden, verzögerten oder kontrollierten Freisetzung solcher Stoffe im Magen.
